Europäisches Patentamt

(19) European Patent Office

Office européen des brevets

(11) Publication number: **0 022 434**
**B1**

# (12) EUROPEAN PATENT SPECIFICATION

(45) Date of publication of patent specification: **28.08.85**

(21) Application number: **80850105.0**

(22) Date of filing: **26.06.80**

(51) Int. Cl.⁴: **C 12 N 11/02**, C 12 P 1/00 // C12P33/06, C12P17/18, C12P15/00, C12N5/00

(54) **Catalysts for the production and transformation of natural products having their origin in higher plants, process for production of the catalysts, and use thereof.**

(30) Priority: **27.06.79 SE 7905615**

(43) Date of publication of application:
**14.01.81 Bulletin 81/02**

(45) Publication of the grant of the patent:
**28.08.85 Bulletin 85/35**

(84) Designated Contracting States:
**AT BE CH DE FR GB IT LI LU NL SE**

(56) References cited:
**DE-A-2 225 039**
**GB-A-1 257 263**

**CHEMICAL ABSTRACTS, vol. 63, 1965, abstract 19001b Columbus, Ohio, US BIOTECHNOLOGY AND BIOENGINEERING, vol. XIX, pages 387-397, 1977 New York, US M. KIERSTAN et al.: "The Immobilization of Microbial Cells, Subcellular Organelles, and Enzymes in Calcium Alginate Gels"**

(73) Proprietor: **Brodelius, Peter**
**Avd. för tillämpad biokemi Kemicentrum Box 740**
**S-220 07 Lund 7 (SE)**

(73) Proprietor: **Mosbach, Klaus**
**Biokemi 2, Kemicentrum Box 740**
**S-220 07 Lund (SE)**

(73) Proprietor: **Zenk, Meinhard**
**Lehrstuhl für Pflanzenphysiologie Ruhr-Universität, Bochum Gebäude ND Universitätsstrasse 150 Postfach 10148 D-4630 Bochum-Querenburg (DE)**

(73) Proprietor: **Deus, Brigitte**
**Lehrstuhl für Pflanzenphysiologie Ruhr-Universität, Bochum Gebäude ND Universitätsstrasse 150 Postfach 10148 D-4630 Bochum-Querenburg (DE)**

(72) Inventor: **Brodelius, Peter**
**Avd. för tillämpad biokemi Kemicentrum Box 740**
**S-220 07 Lund 7 (SE)**

Inventor: **Mosbach, Klaus**
**Biokemi 2, Kemicentrum Box 740**
**S-220 07 Lund (SE)**

Courier Press, Leamington Spa, England.

(56) References cited:
**CHEMICAL ABSTRACTS, vol. 86, no. 3, 17th January 1977, page 201, abstract 13894v Columbus, Ohio, US J. STOECKIGT et al.: "Synthesis of ajmalicine and related indole alkaloids by cell free extracts of Catharanthus roseus cell suspension cultures"**
**CHEMICAL ABSTRACTS, vol. 87, no. 17 24th October 1977, page 355, abstract 130470k Columbus, Ohio, US T.I. ANDREEVA: "Indole alkaloids of a callus culture of Vinca rosea"**
**CHEMICAL ABSTRACTS, vol. 91, no. 10, 3rd September 1979, page 440, abstract 87596z Columbus, Ohio, US P. BRODELIUS et al.: "Immobilized plant cells for the production and transformation of natural products"**

(72) Inventor: **Zenk, Meinhard**
**Lehrstuhl für Pflanzenphysiologie Ruhr-Universität, Bochum Gebäude ND Universitätsstrasse 150 Postfach 10148 D-4630 Bochum-Querenburg (DE)**
Inventor: **Deus, Brigitte**
**Lehrstuhl für Pflanzenphysiologie Ruhr-Universität, Bochum Gebäude ND Universitätsstrasse 150 Postfach 10148 D-4630 Bochum-Querenburg (DE)**

(74) Representative: **Weber, Manfred, Dr. et al**
**Boehringer Mannheim GmbH Patentabteilung Sandhofer Strasse 116 D-6800 Mannheim 31 (DE)**

2

## Description

The present invention relates to immobilized biocatalysts for the production and transformation of natural products having their origin in higher plants, a process for the production of the immobilized biocatalysts and the use thereof in the production or transformation of natural products having their origin in higher plants.

Higher plants are not only the most important sources of foods, oils, fibres and wood, but also a plentiful source of substances utilized for the production of pharmaceuticals and as food additives. The production of many important pharmaceuticals is therefore dependent upon raw materials extracted from in many cases tropical plants, and is therefore heavily dependent upon distant producers. Alternative production methods are therefore of great interest in order to avoid supply shortages. There is also a shortage of certain natural products, which could however be covered if alternative production methods were available.

Moreover, the substances utilized in the production of pharmaceuticals and as food additives mostly are secondary metabolites, for which reason they are often present in very low concentrations in the plant tissue. As a consequence large amounts of plant material are required for the isolation of a given substance in substantial amounts. Nevertheless, a great many pharmaceuticals. are at present produced from higher plants, such as steroids (from diosgenin), codeine, atropine, reserpine, hyoscyamine, digoxin, scopolamine, digitoxin, pilocarpine, quinidine, colcinine, emetine, morphine, quinine, sennosides, tubocurarine, vincristine (see Farmsworth and Morrins (1976), Am. J. Pharmacy 148, p. 46-52 and WHO 1978).

By the present invention, bio catalysts for the production of transformation of important natural products having their origin in higher plants are provided by immobilization. The advantages associated with the use of these immobilized biocatalysts for the production or transformation of natural products reside int. al. in lower costs because of the possibility of a reuse of the biocatalyst and because there is no need for separation of the product from the biocatalyst. Further, immobilized biocatalysts are ideally suited for flow-through processes. Besides, the chemical potential for normally slow growing plant cells (in batch processes) can be exploited more efficiently by the immobilization.

It is prior art to immobilize cells of microorganisms in different ways and also to cultivate animal cells on polymeric materials. A prerequisite of animal cells often is that the cells are absorbed on a carrier to be able to survive outside the organism. On the other hand, it is very surprising that cells isolated from cell cultures of higher plants which compared to microorganisms are extremely large, survive in immobilized state and above all that the metabolism of the isolated cells remains intact.

Subject matter of the invention are immobilized biocatalysts for production or transformation of natural products having their origin in higher plants, characterized in that they comprise

I) particles of a polymer in the pores or network of which there are entrapped and/or absorbed and/or covalenty bound biocatalysts or
II) particles of crosslinked biocatalysts,

said biocatalysts being protoplasts isolated from higher plants, or whole cells, protoplasts, protoplasts with regenerated cell walls, isolated from cell cultures of higher plants.

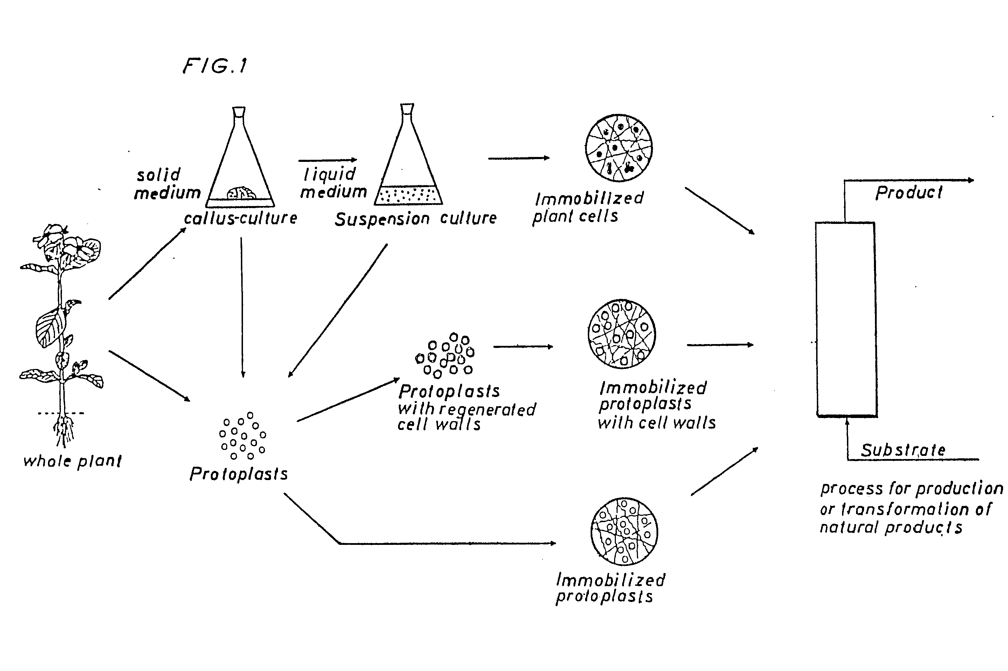

The current biocatalysts thus are immobilized protoplasts isolated from higher plants, or whole cells, protoplasts, protoplasts with regenerated cell walls, isolated from cell cultures, preferably callus and suspension cultures, of higher plants. The cell material is immobilized for example by being entrapped and/or absorbed and/or covalently bound in the pores or network of a porous polymer or by being crosslinked with a suitable crosslinking agent (Fig. 1). In this manner, a readily handled biocatalyst is obtained, which can be used for the production of primary or secondary metabolites de novo or from suitable precursors or for the transformation of metabolites.

The biotransformation according to the invention is better than chemical transformation with regard to yield and secondary products (no by products are formed since the biotransformation is specific).

In most cases the desired product is stored within the cells when production takes place in free cells. For immobilized cells the product in certain cases, however, spontaneously leaks out into the surrounding medium, which can be exploited in a continuous process. This does not occur with the corresponding non-immobilized cells. In other cases the plant cells may be treated after immobilization with surfactants (for example DMSO, toluene, chloroform, cetyltrimethylammoniumbromide, Triton, Tween, SDS, etc). which alter the permeability of the cell membrane so that the product leaks out continuously. In this procedure also, great advantages are associated with immobilized cells, since these treated cells are favourably stabilized by the surrounding polymeric matrix.

The immobilized cells in many cases produce considerably much larger amounts of the desired product per cell than do cells under the same conditions in free suspension. This increased production can be further increased, or an increased production can be induced by placing the immobilized cells in a nutrient so modified that the growth of cells is inhibited. Non-inhibited growth of the immobilized cells can result in bursting of the polymeric particle in which the cells are immobilized. By hormone-limiting conditions at the production an inhibited growth thus results in a drastically increased yield. Further, phosphate is an important nutrient for growing plant cells. By phosphate, and also sulphate, limitation the growth of cells is inhibted, which results in an increased synthesis of secondary metabolites. In this case, too, the availability of nitrogen and carbon sources under growth-limiting conditions would seem to lead to an increased rate of synthesis of alkaloids and other secondary products. By subjecting the cells to a state of stress with the aid of a hypertonic medium growth is likewise inhibited, which again leads to an increased yield of alkaloids and other secondary products. It is remarkable that this induced synthesis of the desired product is so much more pronounced in immobilized cells.

Suitable polymers in the pores or network of which the biocatalyst entities are entrapped and/or adsorbed and/or covalently bound, are for example polysaccharides, preferably alginate or carragheenan, synthetic polymers or crosslinked polyamines. Immobilization proceeds for example in such a manner that a solution or suspension of a polysaccharide or a polymerizable monomer is caused to polymerize in the presence of the cell material. A polyvalent polysaccharide, preferably alginate or carragheenan, is caused to polymerize by contacting a solution or suspension of the polysaccharide with a salt solution containing mono- or divalent cations. For immobilization of for example the cell material by means of a synthetic polymer, one polymerizes say a vinyl monomer, such as an acryl monomer, in the presence of the cell material under normal conditions. As mentioned above, one can also use as network structure, a polyamine, preferably an inert protein, such as albumin, collagen or gelatine, said polyamine being crosslinked with a di- or polyaldehyde, preferably glutaraldehyde. Polysaccharides also, such as chitin, can be crosslinked with a di- or polyaldehyde and then used as network structure.

Finally, the biocatalyst itself can be caused to aggregate by means of a crosslinking agent, such as glutaraldehyde, imidoester or triazine. Examples of imidoesters are dimethyladipimidate, diethyl-3,3'-dithiolbispropionimidate, dimethylpimelimidate, dimethylsuberimidate, and examples of triazines are 1,3-dichloro-5-methoxytriazine, 1,3,5-trichlorotriazine.

The biocatalysts according to the invention can thus be used for production or transformation of primary or secondary metabolites, said metabolites being contacted with a substrate solution in a continuous or discontinuous reactor. The substrate solution preferably is a medium containing carbon and nitrogen sources for de novo synthesis, precursors for partial synthesis, or the substance to be transformed.

As the product spontaneously leaks from the immobilized cells the biocatalyst can be used for continuous production, preferably in a column reactor. If need be, the cells may, as earlier described, be treated with a surfactant so that the permeability of the cell membrane is altered. After such a treatment the biocatalyst can be utilized for synthesis in a continuous reactor.

Thus, a substrate solution is recycled through the reactor, and the released metabolities are continuously extracted from the recycling substrate solution. Alternatively, a substrate solution can be pumped through the reactor without recycling. The released products in this case can later be isolated from the eluate of the reactor. In discontinuous operation use is made in most cases of a simple batch reactor.

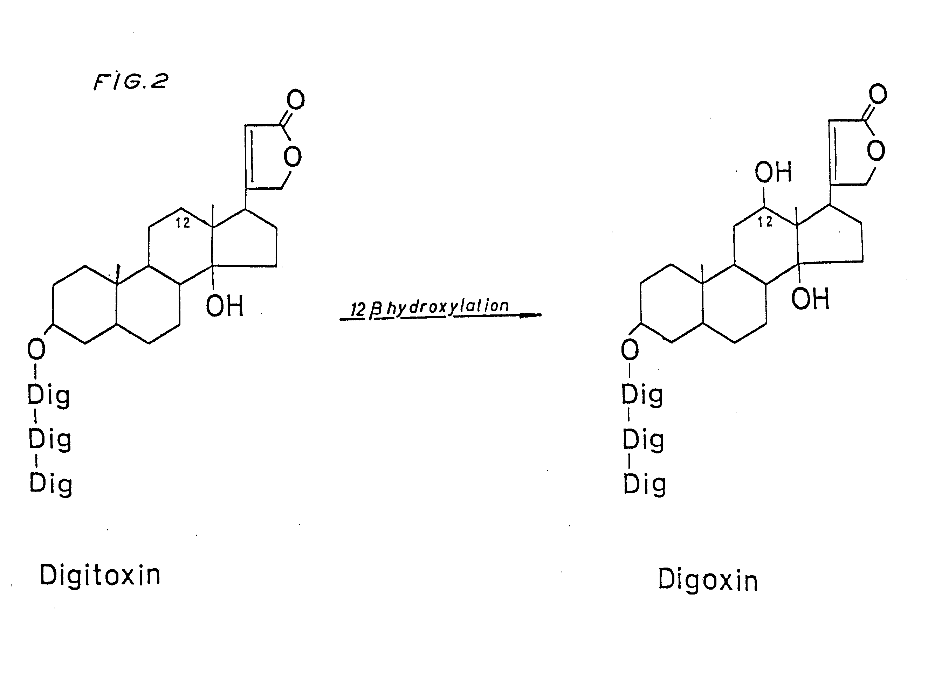

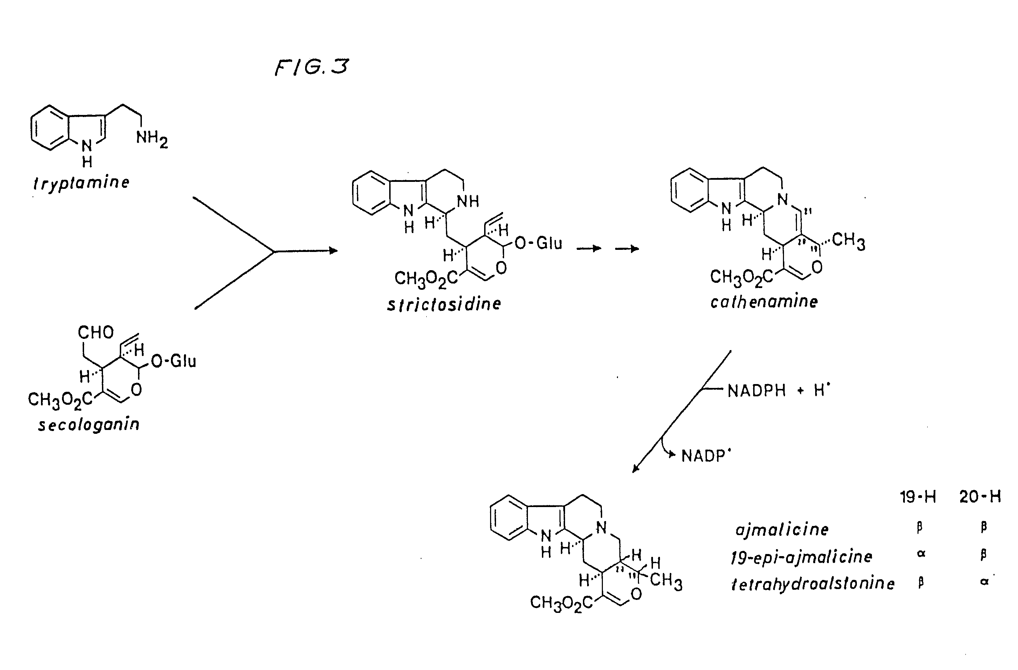

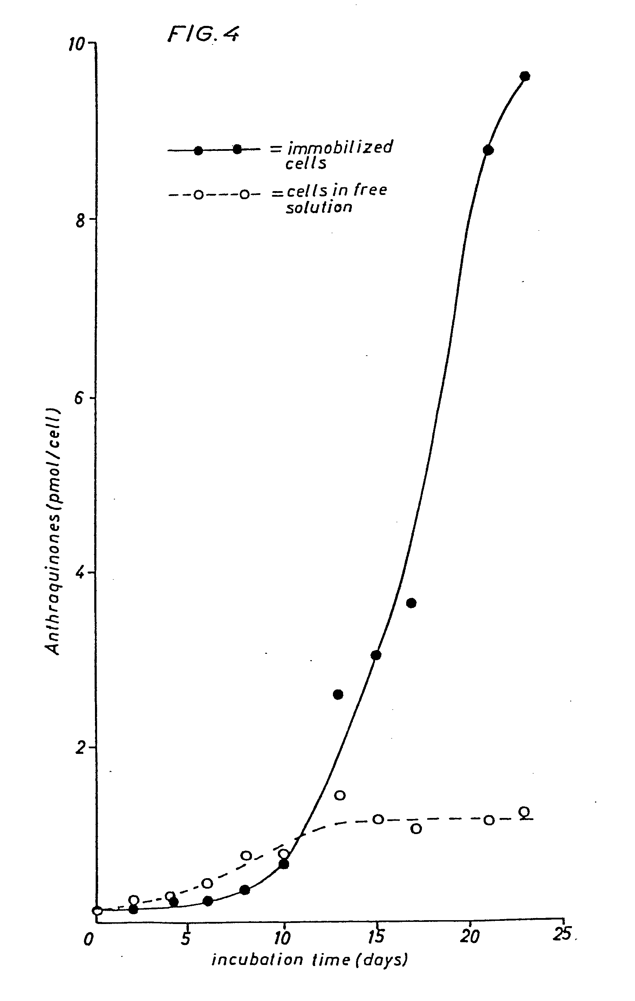

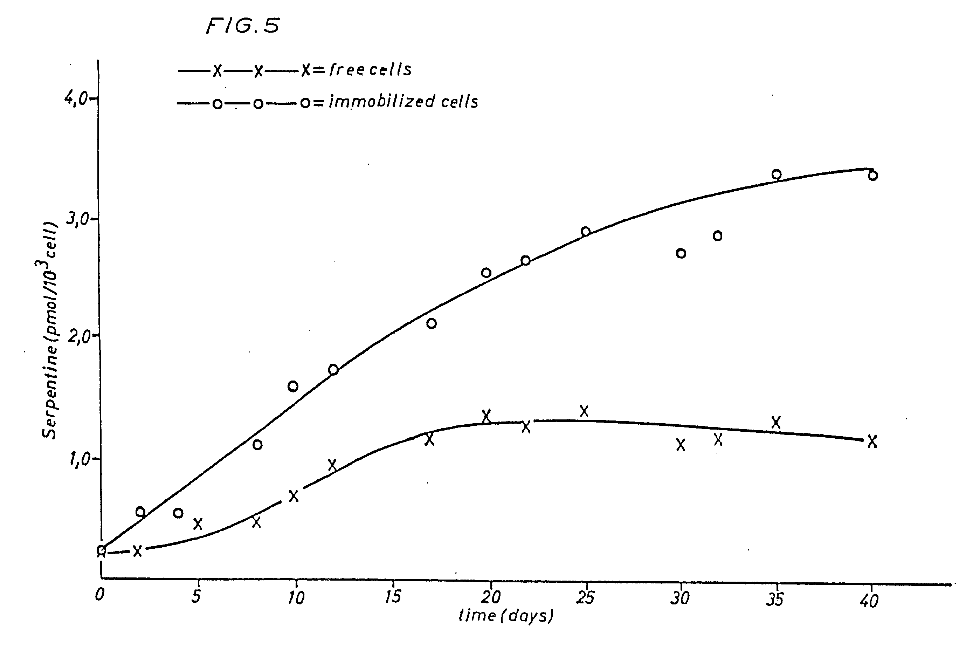

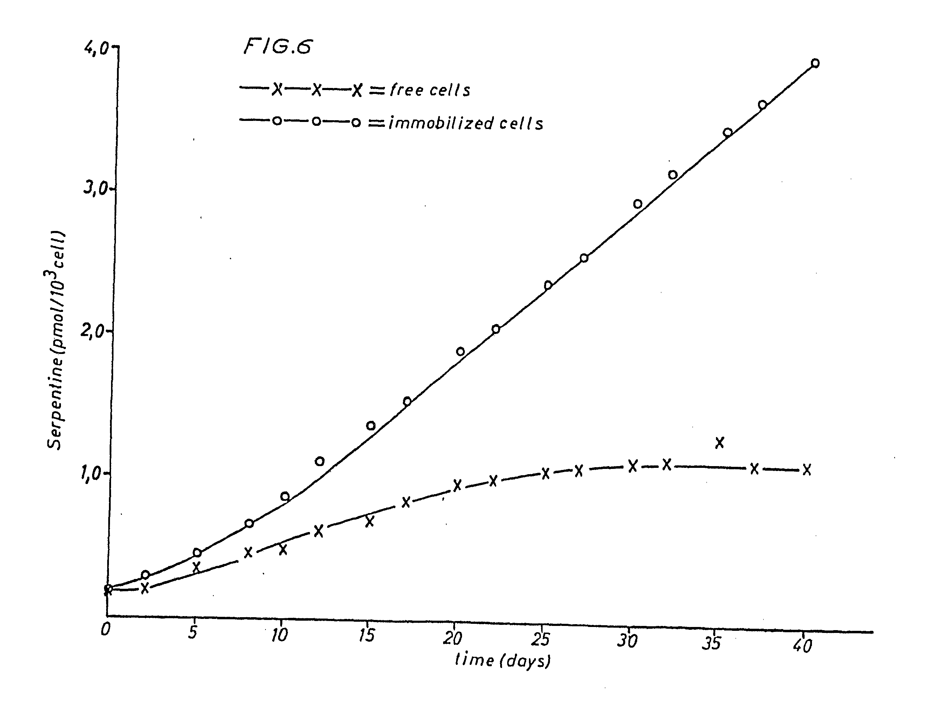

Some specific examples of the use of immobilized cells isolated from cell cultures of higher plants are the hydroxylation of digitoxin to digoxin using immobilized cells of Digitalis lanata (Fig. 2) in batch processes or in continuous processes.

Other A-series cardiac glycosides can also be ß-hydroxylated in a similar manner. The following table gives examples of A-glycosides which are ß-hydroxylated to the corresponding C-glycosides.

#### TABLE

| A-glycosides | C-glycosides |
| --- | --- |
| digitoxin | digoxin |
| β-acetyldigitoxin | β-acetyldigoxin |
| α-acetyldigitoxin | α-acetyldigoxin |
| β-methyldigitoxin | β-methyldigoxin |
| α-methyldigitoxin | α-methyldigoxin |
| purpureaglycoside-A | desacetyl-lanatoside-C |
| lanatoside-A | lanatoside-C |

A further result obtained with the use of immobilized plant cells according to the present invention is that the productive phase of the cells during incubation is prolonged, whereby they can be utilized for a much longer time for production of the desired product.

For a better understanding of the invention, reference is made hereinbelow to the accompanying drawings in which

Fig. 1 shows examples of alternative methods of preparing and immobilizing plant cells;

Fig. 2 shows β-hydroxylation of digitoxin to digoxin.

The invention will be elucidated in greater detail by the following examples.

Example 1
Hydroxylation of digitoxin to digoxin using immobilized cells of Digitalis lanata, batch process

Cells of *Digitalis lanata* (3 ml) cultivated in suspension culture were suspended in sterile 3% Na-alginate (10 ml) and immobilized by dripping the solution into a sterile nutrient solution containing $CaCl_2$ (0.05 M). The immobilized cells were placed under sterile conditions in an E-flask containing nutrient solution according to Nitsch and Nitsch (25 ml) with an addition of digitoxin (500 μg). The immobilized cells hydroxylated the added digitoxin specifically in 12-position to digoxin. The hydroxylation proceeded for at least 33 days (Fig. 2).

Example 2
Hydroxylation to digitoxin in digoxin using immobilized digitalis cells in a continuous column reactor

Digitalis cells were obtained by cultivation in suspension cultures which had been established from plants of *Digitalis lanata* with a much higher than average 12-hydroxylation power.

Cells having an increased hydroxylation power were immobilized in a manner analogous to that described in Example 1. Under sterile conditions the beads containing immobilized digitalis cells were filled into a column (3×20 cm). Digitoxin ($5 \cdot 10^{-5}$ M) in an aqueous solution, pH 5.7, was pumped under sterile conditions through the column at a rate of 22 ml/h. The hydroxylation of digitoxin to digoxin (maximum yield 77%) could be observed by radioimmunotests. Even after 70 days of continuous operation the column showed between 70 and 80% of the maximum hydroxylation capacity.

Example 3
Immobilization of protoplasts of *catharanthus roseus*

Protoplasts were prepared from intact plant tissue, for example leaves of *Catharanthus roseus* or from callus or suspension cultures of the same species. The protoplasts obtained were mixed with isotonic Na-alginate solution and the suspension was then dripped into an isotonic $CCl_2$ solution. The immobilized protoplasts can then directly be used for production or transformation of natural products, alternatively a regeneration of the cell wall can be allowed to take place in a suitable nutrient solution.

**Claims**

1. Immobilized biocatalysts for production or transformation of natural products having their origin in higher plants, characterized in that they comprise

I) particles of a polymer in the pores or network of which there are entrapped and/or absorbed and/or covalenty bound biocatalysts or
II) particles of crosslinked biocatalysts,

said biocatalysts being protoplasts isolated from higher plants, or whole cells, protoplasts, protoplasts with regenerated cell walls, isolated from cell cultures of higher plants.

2. Immobilized biocatalysts according to claim 1, characterised in that the cell cultures of higher plants are callus or suspension cultures.

3. Immobilized biocatalysts according to claim 1 or 2, characterised in that the polymer is a polysaccharide, an acryl polymer or a crosslinked polyamine.

4. Immobilized biocatalysts according to claim 3, characterised in that the polysaccharide is alginate or carragheenan.

5. Immobilized biocatalysts according to claim 3, characterised in that the crosslinked polyamine is albumin, collagen or gelatine crosslinked with glutaraldehyde.

6. Process of producing immobilized biocatalysts according to claim 1, characterised by

a) polymerizing a solution or suspension of polysaccharide with a salt solution containing mono- or divalent cations in the presence of said biocatalysts, or

b) polymerizing a solution or suspension of polysaccharide with glutaraldehyde as crosslinking agent in the presence of said biocatalysts, or

c) polymerizing a solution or suspension of synthetic monomer in the presence of said biocatalysts, or

d) polymerizing a polyamin with glutaraldehyde as crosslinking agent in the presence of said biocatalysts, or

e) aggregating said biocatalyst itself by means of glutaraldehyde, imidoester or triazine.

7. A process according to claim 6, characterised in that the immobilized biocatalysts are treated with a surfactant selected from the group consisting of dimethylsulfoxide and chloroform to change the permeability of the cell membrane.

8. Use of immobilized biocatalysts according to claim 1 for production or transformation of natural products having their origin in higher plants, characterised in that the biocatalysts are contacted with a substrate solution in a continuous or discontinuous reactor, said solution being a medium containing

a) carbon and nitrogen sources for de novo synthesis, or

b) precursors for partial syntheses or

c) the substrate to be transformed.

9. Use according to claim 8, characterised in that the production or transformation is performed under hormone-limiting conditions, whereby the immobilized biocatalysts form the desired product to a higher extent than do the corresponding non-immobilized biocatalysts.

10. Use according to claim 8, characterised in that the production or transformation is performed under phosphate- or sulphate-limiting conditions, whereby the immobilized biocatalysts form the desired product to a higher extent than do the corresponding non-immobilized biocatalysts.

11. Use according to claim 8, characterised in that the immobilized biocatalysts are subjected to a state of stress by means of a hypertonic medium, whereby cell growth is inhibited and the immobilized biocatalysts form the desired product to a higher extent than do the corresponding non-immobilized biocatalysts.

12. Use according to one or more of claims 8—11, characterised in that the immobilized biocatalysts spontaneously release the desired product into the surrounding medium.

13. Use according to one or more of claims 8—11 characterised in that the substrate solution is recycled through the reactor which contains the immobilized biocatalysts the cell membrane permeability of which has been changed by treatment with a surfactant, whereby released metabolites are continuously extracted from the substrate solution.

## Patentansprüche

1. Immobilisierte Biokatalysatoren für die Herstellung oder Umwandlung natürlicher Produkte, die ihren Ursprung in höheren Pflanzen haben, dadurch gekennzeichnet, dass sie

I) Partikel eines Polymeren, in dessen Poren oder Netzwerk Biokatalysatoren eingeschlossen und/oder absorbiert und/oder kovalent gebunden sind, oder

II) Partikel von vernetzten Biokatalysatoren aufweisen, wobei die Biokatalysatoren aus höheren Pflanzen isolierte Protoplasten oder aus Zellkulturen von höheren Pflanzen isolierte ganze Zellen, Protoplasten oder Protoplasten mit regenerierten Zellwänden sind.

2. Immobilisierte Biokatalysatoren nach Anspruch 1, dadurch gekennzeichnet, dass die Zellkulturen von höheren Pflanzen Kallus- oder Suspensionskulturen sind.

3. Immobilisierte Biokatalysatoren nach Anspruch 1 oder 2, dadurch gekennzeichnet, dass das Polymer ein Polysaccharid, ein Acrylpolymer oder ein vernetztes Polyamin ist.

4. Immobilisierte Biokatalysatoren nach Anspruch 3, dadurch gekennzeichnet, dass das Polysaccharid Alginat oder Carrageenan ist.

5. Immobilisierte Biokatalysatoren nach Anspruch 3, dadurch gekennzeichnet, dass das vernetzte Polyamin mit Glutaraldehyd vernetztes Albumin oder Kollagen oder mit Glutaraldehyd vernetzte Gelatine ist.

6. Verfahren zur Herstellung von immobilisierten Biokatalysatoren nach Anspruch 1, dadurch gekennzeichnet, dass man

a) eine Polysaccharidlösung oder -suspension mit einer ein- oder zweiwertige Kationen enthaltenden Salzlösung in Gegenwart der Biokatalysatoren polymerisiert oder

b) eine Polysaccharidlösung oder -suspension mit Glutaraldehyd als Vernetzungsmittel in Gegenwart der Biokatalysatoren polymerisiert oder

c) eine Lösung oder Suspension von synthetischem Monomer in Gegenwart der Biokatalysatoren polymerisiert oder

d) ein Polyamin mit Glutaraldehyd als Vernetzungsmittel in Gegenwart der Biokatalysatoren polymerisiert oder

e) den Biokatalysator selbst mit Hilfe von Glutaraldehyd, Imidoester oder Triazin aggregiert.

7. Verfahren nach Anspruch 6, dadurch gekennzeichnet, dass man die immobilisierten Biokatalysatoren mit einem Tensid behandelt, das aus der Gruppe gewählt ist, die aus Dimethylsulfoxyd und Chloroform besteht, um die Permeabilität der Zellmembran zu verändern.

8. Verwendung von immobilisierten Biokatalysatoren nach Anspruch 1 zur Herstellung oder Umwandlung natürlicher Produkte, die ihren Ursprung in höheren Pflanzen haben, dadurch gekennzeichnet, dass man die Biokatalysatoren in einem kontinuierlichen oder diskontinuierlichen Reaktor mit einer Substratlösung in Berührung bringt, wobei die Lösung ein Nährmedium ist, das

a) Kohlenstoff- und Stickstoffquellen für die de novo-Synthese oder

b) Vorstufen für Partialsynthesen oder

c) die umzuwandelnde Substanz enthält.

9. Verwendung nach Anspruch 8, dadurch gekennzeichnet, dass man die Herstellung oder Umwandlung unter hormonregulierenden Bedingungen ausführt, wodurch die immobilisierten Biokatalysatoren das gewünschte Produkt in einem höheren Ausmass bilden als die entsprechenden nichtimmobilisierten Biokatalysatoren.

10. Verwendung nach Anspruch 8, dadurch gekennzeichnet, dass man die Herstellung oder Umwandlung unter phosphat- oder sulfatregulierenden Bedingungen ausführt, wodurch die immobilisierten Biokatalysatoren das gewünschte Produkt in einem höheren Ausmass bilden als die entsprechenden nichtimmobilisierten Biokatalysatoren.

11. Verwendung nach Anspruch 8, dadurch gekennzeichnet, dass man die immobilisierten Biokatalysatoren mit Hilfe eines hypertonischen Mediums einem Stresszustand aussezt, wodurch das Zellwachstum gehemmt wird und die immobilisierten Biokatalysatoren das gewünschte Produkt in einem höheren Ausmass bilden als die entsprechenden nichtimmobilisierten Biokatalysatoren.

12. Verwendung nach einem oder mehreren der Ansprüche 8 bis 11, dadurch gekennzeichnet, dass die immobilisierten Biokatalysatoren das gewünschte Produkt spontan in das umgebende Medium freisetzen.

13. Verwending nach einem oder mehreren der Ansprüche 8 bis 11, dadurch gekennzeichnet, dass man die Substratlösung im Kreislauf durch den Reaktor führt, der die immobilisierten Biokatalysatoren enthält, deren Zellmembranpermeabilität durch Behandlung mit einem Tensid verändert worden ist, wodurch die freigesetzten Metaboliten kontinuierlich aus der Substratlösung extrahiert werden.

## Revendications

1. Biocatalyseurs immobilisés pour la fabrication ou la transformation de produits naturels provenant de plantes supérieures, caractérisés en ce qu'ils comprennent:

I) des particules d'un polymère dans les pores ou le réseau duquel sont inclus et/ou absorbés et/ou liés par covalence, des biocatalyseurs, ou,

II) des particules de biocatalyseurs réticulés,

ces biocatalyseurs étant des protoplastes isolés à partir de plantes supérieures, ou des cellules entières, des protoplastes, des protoplastes avec des parois cellulaires régénérées, isolés à partir de cultures de cellules de plantes supérieures.

2. Biocatalyseurs immobilisés selon la revendication 1, caractérisé en ce que le cultures de cellules de plantes supérieures sont des cultures de cal ou des cultures en suspension.

3. Biocatalyseurs immobilisés selon la revendication 1 ou 2, caractérisés en ce que le polymère est un polysaccharide, un polymère acrylique ou une polyamine réticulée.

4. Biocatalyseurs immobilisés selon la revendication 3, caractérisés en ce que le polysaccharide est un alginate ou le carraghen.

5. Biocatalyseurs immobilisés selon la revendication 3, caractérisés en ce que le polyamine réticulée est l'albumine, le collagène ou la gélatine réticulée par du glutaraldéhyde.

6. Procédé de préparation de biocatalyseurs immobilisés selon la revendication 1, caractérisé en ce que:

a) on polymérise une solution ou une suspension d'un polysaccharide au moyen d'une solution saline contenant des cations mono- ou divalents en présence des biocatalyseurs, ou,

b) on polymérise une solution ou une suspension d'un polysaccharide avec du glutaraldéhyde comme agent réticulant en présence des biocatalyseurs, ou,

c) on polymérise une solution ou une suspension d'un monomère synthétique en présence des biocatalyseurs, ou,

d) on polymérise une polyamine avec du glutaraldéhyde comme agent de réticulation en présence des biocatalyseurs, ou,

e) on fait s'agglutiner le biocatalyseur lui-même au moyen de glutaraldéhyde, d'un imido-ester ou de triazine.

7. Procédé selon la revendication 6, caractérisé en ce que les biocatalyseurs immobilisés sont traités par un surfactant choisi dans le groupe comprenant le diméthylsulfoxyde et le chloro-

forme, pour modifier la perméabilité de la membrane cellulaire.

8. Utilisation de biocatalyseurs immobilisés selon la revendication 1, pour la fabrication ou la transformation de produits naturels provenant de plantes supérieures, caractérisée en ce que les biocatalyseurs sont mis en contact avec une solution de la matière à traiter dans un réacteur à fonctionnement continu ou discontinu, cette solution étant un milieu contenant:

a) des sources de carbone et d'azote pour la synthèse complète, ou,

b) des produits précurseurs pour des synthèses partielles, ou

c) la substance à transformer.

9. Utilisation selon la revendication 8, caractérisée en ce que la fabrication ou la transformation est effectuée dans des conditions limitant la présence d'hormones, dans lesquelles les biocatalyseurs immobilisés forment le produit recherché à un taux plus élevé que ne le font les biocatalyseurs non immobilisés correspondants.

10. Utilisation selon la revendication 8, caractérisée en ce que la fabrication ou la transformation est effectuée dans des conditions limitant la présence de phosphates ou de sulfates, dans lesquelles les biocatalyseurs immobilisés forment le produit recherché à un taux plus élevé que ne le font les biocatalyseurs non immobilisés correspondants.

11. Utilisation selon la revendication 8, caractérisée en ce que les biocatalyseurs immobilisés sont soumis à un état de tension au moyen d'un milieu hypertonique, dans lequel la croissance cellulaire est inhibée et les biocatalyseurs immobilisés forment le produit recherché à un taux plus élevé que ne le font les biocatalyseurs non immobilisés correspondants.

12. Utilisation selon une ou plusieurs des revendications 8 à 11, caractérisée en ce que les biocatalyseurs immobilisés libèrent le produit recherché spontanément dans le milieu environnant.

13. Utilisation selon une ou plusieurs des revendications 8 à 11, caractérisée en ce que la solution de la matière à traiter est recyclée dans le réacteur contenant les biocatalyseurs immobilisés dont la perméabilité de la membrane cellulaire a été modifiée par le traitement par un surfactant, les métabolites libérés au cours de ce dernier étant extraits en continu de la solution de la matière à traiter.

FIG.1

solid medium

callus-culture

liquid medium

Suspension culture

Immobilized plant cells

whole plant

Protoplasts

Protoplasts with regenerated cell walls

Immobilized protoplasts with cell walls

Immobilized protoplasts

Product

Substrate

process for production or transformation of natural products

0 022 434

FIG.2

12 β hydroxylation

Digitoxin

Digoxin

FIG.3

tryptamine

secologanin

strictosidine

cathenamine

—NADPH + H⁺

↳ NADP⁺

|  | 19-H | 20-H |
| --- | --- | --- |
| ajmalicine | β | β |
| 19-epi-ajmalicine | α | β |
| tetrahydroalstonine | β | α |

FIG.4

Anthraquinones (pmol/cell)

incubation time (days)

●——● = immobilized cells

--o----o-- = cells in free solution

4

FIG.5

—x——x— x = free cells
—o——o— o = immobilized cells

Serpentine (pmol/10³ cell)

time (days)

5

FIG.6

—x—x—x = free cells
—o—o—o = immobilized cells

Serpentine (pmol/10³ cell) vs. time (days)